## Europäisches Patentamt

## European Patent Office

## Office européen des brevets

(11) Veröffentlichungsnummer: **0 279 955**
**B1**

(12) **EUROPÄISCHE PATENTSCHRIFT**

(45) Veröffentlichungstag der Patentschrift:
04.07.90

(51) Int. Cl.⁵: **G03B 42/04**

(21) Anmeldenummer: **87119359.5**

(22) Anmeldetag: **30.12.87**

(54) **Filmhalter für endodontische Zahnaufnahmen.**

(30) Priorität: **28.01.87 DE 8701308 U**

(43) Veröffentlichungstag der Anmeldung:
**31.08.88 Patentblatt 88/35**

(45) Bekanntmachung des Hinweises auf die Patenterteilung:
**04.07.90 Patentblatt 90/27**

(84) Benannte Vertragsstaaten:
**AT BE CH DE ES FR GB GR IT LI LU NL SE**

(56) Entgegenhaltungen:
**DE-U- 8 610 962**
**US-A- 4 592 084**
**US-A- 4 593 401**

(73) Patentinhaber: **Fuhrmann, Andreas, Dr. med.,**
**Bellmannstrasse 32, D-2000 Hamburg 52(DE)**

(72) Erfinder: **Fuhrmann, Andreas, Dr. med.,**
**Bellmannstrasse 32, D-2000 Hamburg 52(DE)**

(74) Vertreter: **Richter, Werdermann & Gerbaulet, Neuer Wall 10, D-2000 Hamburg 36(DE)**

## Beschreibung

Die Erfindung bezieht sich auf einen Filmhalter für endodontische Zahnaufnahmen in der Parallel-Technik, der aus einem als Formkörper ausgebildeten Aufbißblock, einem an diesem angeformten und senkrecht zu der vom Aufbißblock gebildeten Aufbißebene stehenden Rückschild zur Halterung eines Zahnfilms, wobei der obere Rand des Rückschildes in Richtung zu dem Formkörper unter Ausbildung einer Zahnfilmhaltenut leicht abgebogen ausgebildet ist, einer mittels einer lösbaren Steckhalterung an dem Aufbißblock gehaltenen Führungsstange und einem auf dieser in Führungsstangenlängsrichtung verschieblichen Visierring mit einer Halterung zum Befestigen des Filmhalters an dem Langtubus eines zahnärztlichen Röntgengerätes, besteht, wobei das Rückschild in eine dem Formkörper zugekehrt angeordnete untere Zahnfilmauflagefläche übergehend ist und gegenüber den Abmessungen des Zahnfilms größere Abmessungen aufweist.

Zur Darstellung des Zahnes werden in der Endodontie Röntgenaufnahmen unter Verwendung von Röntgenapparaten, die für zahnärztliche Zwecke ausgebildet sind, gemacht, wobei die Längenmessung des Wurzelkanals in der Endodontie von großer Bedeutung ist. Diese Längenmessung wird mit Hilfe von Zahnfilmaufnahmen ermittelt. Als ideale Aufnahmetechnik für Meßaufnahmen findet die Parallel-Technik Anwendung. Bei dieser Röntgen-Aufnahmetechnik ist der Zentralstrahl rechtwinklig auf die Zahnachse und die Filmebene gerichtet. Für intraorale Aufnahmen sind Zahnfilme entwickelt worden, die plattenförmig ausgebildet sind und genormte Abmessungen aufweisen, so daß ein Einführen des Zahnfilms in die Mundhöhle möglich ist. In Abhängigkeit von der Lage des jeweiligen Zahnes, der aufgenommen werden soll, ist das Zahnfilmplättchen nach Einbringen in den Mund des Patienten vom Patienten selbst in der jeweils erforderlichen Stellung zu halten, was durch Andrücken des Zahnfilms mittels Daumen oder Zeigefinger gegen das aufzunehmende Objekt erfolgt.

Insbesondere zur Anwendung der Parallel-Technik ist ein Filmhalter mit den voranstehend angegebenen Merkmalen entwickelt worden, mit dem der Film so in den Mund des Patienten gebracht wird, daß er parallel zur Längsachse des Zahnes steht. Wegen der Platzverhältnisse im Mund muß der Film an manchen Stellen einen größeren Abstand vom Zahn haben und damit keine Verzerrungen entstehen, muß dann auch der Fokus-Film-Abstand vergrößert werden, was mittels des verlängerten Tubus an der Röntgenröhre, d.h. an dem Röntgengerät, vorgenommen wird.

Bei derartigen Filmhaltern wird der Film an einem Rückschild gehalten, an dem eine Bißplatte angeformt ist, vermittels der der Filmhalter vom Patienten vermittels Zahndrucks gehalten wird. Derartige Filmhalter sind jedoch für Meßaufnahmen mit Wurzelkanalinstrumenten und/oder Cofferdam nicht geeignet. Aus diesem Grunde werden endodontische Aufnahmen in der Regel mit der Halbwinkel-Technik hergestellt, bei der es sich um eine Röntgen-Aufnahmetechnik handelt, bei der der Zentralstrahl auf den Apex gerichtet senkrecht auf die Winkelhalbierende, zwischen der Filmebene und der Zahnachse gerichtet ist. Diese Halbwinkel-Technik führt jedoch oftmals zu unbefriedigenden Ergebnissen, was sowohl für die Längenmessung des Wurzelkanals als auch für die Reproduzierbarkeit gilt, denn die verschiedenen Kontrollaufnahmen einer Wurzelbehandlung können nur schwer miteinander verglichen werden. Hinzu kommt, daß die mittig zu dem Rückschild des Filmhalters angeordneten Bißplatten so bemessen und an dem Rückschild angeordnet sind, daß der Einsatz von Wurzelkanalinstrumenten nicht möglich ist, da die zur Wurzelkanalaufbereitung verwendeten Nadeln während der Röntgenaufnahmen im Wurzelkanal verbleiben, wobei das Griffende dieser Nadeln oder Pfeilhalter aus dem Wurzelkanal herausragen und derart im Bereich der Bißplatte eines in dem Mund des Patienten eingebrachten Filmhalters zu liegen kommen, daß die herkömmlichen Filmhalter nicht eingesetzt werden können. Außerdem weisen die Rückschilder der Filmhalter gegenüber den verwendeten Zahnfilmen kleinere Abmessungen auf, so daß der Zahnfilm über den umlaufenden Rand des Rückschildes mit einem Abschnitt herausragt, so daß während der Röntgenaufnahme durch Druckeinwirkung der Film verbogen wird. Ein Abknicken des Zahnfilms führt zu kleinen elektrischen Entladungen in der Emulsionsschicht des Films, die sich auf dem entwickelten Film als schwarze Striche zeigen und zu Mißdeutungen führen können. Des weiteren empfindet der Patient beim Zusammenbeißen der Zähne einen Druckschmerz, hervorgerufen durch die obere Kante des mittels des Filmhalters in den Mund des Patienten eingebrachten Filmes, der mit seinen relativ scharfen Kanten in Kontakt mit der empfindlichen Schleimhaut des Gaumens und es so durch starke Druckeinwirkung zu Schmerzen kommt, so daß der Patient oftmals dazu neigt, dem Schmerz dadurch auszuweichen, daß der Bißdruck verringert wird, was aber wiederum zu einer Lagenverschiebung des Zahnfilmes führen kann, obwohl dieser im Filmhalter gehalten ist.

Durch die DE-U 8 610 962 ist ein Filmhalter für endodontische Zahnaufnahmen in der Parallel-Technik, bestehend aus einer Bißplatte, einem an dieser angeformten und senkrecht zur Bißplatte stehenden Rückschild zur Aufnahme eines Zahnfilms, einer an der Bißplatte ausgebildeten Klemmhalterung für den Zahnfilm an dem Rückschild, einer mittels einer lösbaren Steckhalterung an der Bißplatte gehaltenen Führungsstange und einem auf dieser in Führungsstangenlängsrichtung verschieblichen Visierring mit einer Halterung zum Befestigen des Filmhalters an dem Langtubus eines zahnärztlichen Röntgengerätes, bekannt, der ein Rückschild mit einer Größe aufweist, die den Abmessungen des Zahnfilms entspricht, wobei das Rückschild mit einer oberen, U-förmig in Richtung zu der Bißplatte abgebogenen, den Zahnfilm umschließenden Kante mit einem etwa kreisbogenförmig abgebogenen Profil versehen ist, wobei der an dem Rückschild anliegende Schenkel des U-förmigen Kantenprofils als federnd-elastische Klemmleiste ausgebildet ist. Aufgrund dieser Ausgestaltung des Rückschildes zur Halterung eines Zahnfilms werden zwar schmerzerzeugende Druckstellen im Mund des Patienten durch hervorstehende Filmkanten vermieden, doch läßt es sich nicht verhindern, daß bei abgeknickten oder angeknickten Zahnfilmrändern vom Patienten unwillkürlich Zungenbewegungen durchgeführt werden, die zu einer Lagenveränderung des Filmhalters im Munde führen, wenn kein ausreichender Bißdruck vorhanden ist. Bei diesem Filmhalter ist das Rückschild zur Halterung des Zahnfilmes direkt an dem Aufbißblock befestigt und zu diesem seitlich versetzt, jedoch ist aufgrund dieser Ausgestaltung kein ausreichender Kontakt mit dem Gegenkiefer während der Meßaufnahmen gewährleistet, denn gerade dieser Kontakt mit dem Gegenkiefer trägt zu einem besseren Halt und einer exakteren Lage des Filmhalters im Munde des Patienten während der Meßaufnahmen bei.

Darüber hinaus sind Filmhalter für endodontische Zahnaufnahmen in den verschiedensten Ausführungsformen bekannt. So besteht der Filmhalter nach der US-A 4 593 401 aus einem etwa U-förmigen Formkörper, der in seinem die beiden Schenkel verbindenden Steg mit einer schlitzförmigen Aufnahmeöffnung versehen ist, die zur Aufnahme des Zahnfilms dient. Die Halterung des Zahnfilms erfolgt dabei in der Weise, daß der Zahnfilm mit einem kleinen Abschnitt in diese schlitzförmige Ausnehmung in dem Halter eingeschoben und in diesem Halteschlitz gehalten wird. Das Problem des Abbiegens Abkantens u.dgl. des Zahnfilms wird mit diesem Zahnfilmhalter jedoch nicht gelöst, da keine vollflächige Anlage des Zahnfilms an der Halterung gewährleistet ist. Hinzu kommt, daß mit diesem Filmhalter keine Meßaufnahmen im Oberkiefer und Aufnahmen mit Anwendung der Parallel-Technik möglich sind.

Durch die US-A 4 592 084 ist ein weiterer Filmhalter mit einer Bißplatte bekannt. Bei diesem Filmhalter ist kein Rückschild zur Aufnahme des Zahnfilms vorgesehen. Der Filmhalter ist als U-förmige Klammerhalterung ausgebildet, die den Zahnfilm mit einem Abschnitt hält. Derjenige Bereich des Zahnfilms, der nicht von der Klammer des Filmhalters gehalten ist, ist ungeschützt, so daß ein Abbiegen und eine Lageveränderung des Zahnfilms möglich ist, was zu ungenauen Aufnahmen führen kann.

Der Erfindung liegt die Aufgabe zugrunde, einen Filmhalter der eingangs genannten Art mit einem erhöhten Patientenkomfort für endodontische Meßaufnahmen in der Parallel-Technik zu schaffen, der ausreichenden Platz für die Aufnahme von Wurzelkanalinstrumenten aufweist, eine zusätzliche Stabilisierung am distalen Ende ermöglicht und bei dem eine plane Halterung des Zahnfilmes gewährleistet ist, ohne daß die scharfen Kanten des Zahnfilms mit der empfindlichen Schleimhaut des harten Gaumens in Kontakt kommen. Außerdem soll der Filmhalter auch Meßaufnahmen im Seitenzahnbereich ermöglichen und die volle Zahnfilmfläche soll zu Meßaufnahmen zur Verfügung stehen.

Diese Aufgabe wird durch die im Patentanspruch gekennzeichneten Merkmale gelöst.

Ein derart ausgebildeter Filmhalter zeichnet sich durch folgende wesentliche und vorteilhafte Merkmale aus:

– Platzhalter für Wurzelkanalinstrumente; durch Erhöhung und Verlagerung des Aufbißblockes nach mesial wird Platz für die Wurzelkanalinstrumente geschaffen.

– Zusätzliche Stabilisierung am distalen Ende; der stegartige Kontakthalteblock des Filmhalters am distalen Ende sorgt durch Kontakt mit dem Gegenkiefer für einen besseren Halt und eine exaktere Lage des Filmhalters im Munde eines Patienten während der Meßaufnahmen.

– Vergrößerung des Rückschildes; die Vergrößerung des Rückschildes über das Zahnfilmformat hinaus verhindert, daß der Film verbiegt und durch Druck seine Lage verändert. Außerdem können die scharfen Kanten der Filmverpackung nicht mehr mit der empfindlichen Schleimhaut des harten Gaumens in Kontakt kommen. Da das Rückschild im Gegensatz zum Zahnfilm an der Oberkante verbreitert und abgerundet ist, empfindet der Patient, wenn er zusammenbeißen muß, keinen Schmerz mehr.

– Verlagerung der unteren Fläche des Kontakthalteblockes in eine oberhalb der Ebene, die von der unteren Fläche des Aufbißblockes gebildet wird, liegende Ebene bei konisch sich verjüngendem Übergang des Aufbißblockes zum Kontakthalteblock im Bereich der unteren Kante des Rückschildes; dadurch verbesserte anatomische Anpassung beim Zusammenbiß.

Die gesamte Ausgestaltung des Filmhalters trägt zu einem verbesserten Patientenkomfort bei. Dieser verbesserte Patientenkomfort kommt in erster Linie einer stabileren Lage des Filmhalters im Munde des Patienten zugute, da der Filmhalter neben dem Aufbißblock und dem stegartigen Kontakthalteblock an dem distalen Ende im Rückschild einen weiteren Abstützblock hält, so daß auch Meßaufnahmen im Seitenzahnbereich möglich sind. Dieser Filmhalter trägt sowohl in der Zahnarztpraxis als auch im Klinikbetrieb dazu bei, daß die korrekte Aufbereitung der Wurzelkanäle besser nachweisbar wird, so daß der Erfolg einer endodontischen Behandlung dadurch wesentlich vergrößert und verbessert wird.

Vorteilhafte Ausgestaltungen der Erfindung sind in den Unteransprüchen gekennzeichnet.

Ausführungsbeispiele der Erfindung werden nachstehend anhand der Zeichnung näher erläutert. Es zeigen

Fig. 1 in einer schaubildlichen Ansicht einen Instrumentensatz, bestehend aus einem rechteckigen Langtubus und einem Filmhalter,
Fig. 2 in einer Vorderansicht den Filmhalter,
Fig. 3 in einer Rückansicht den Filmhalter,
Fig. 4 in einer Ansicht von oben den Filmhalter und
Fig. 5 in einer Seitenansicht den Filmhalter.

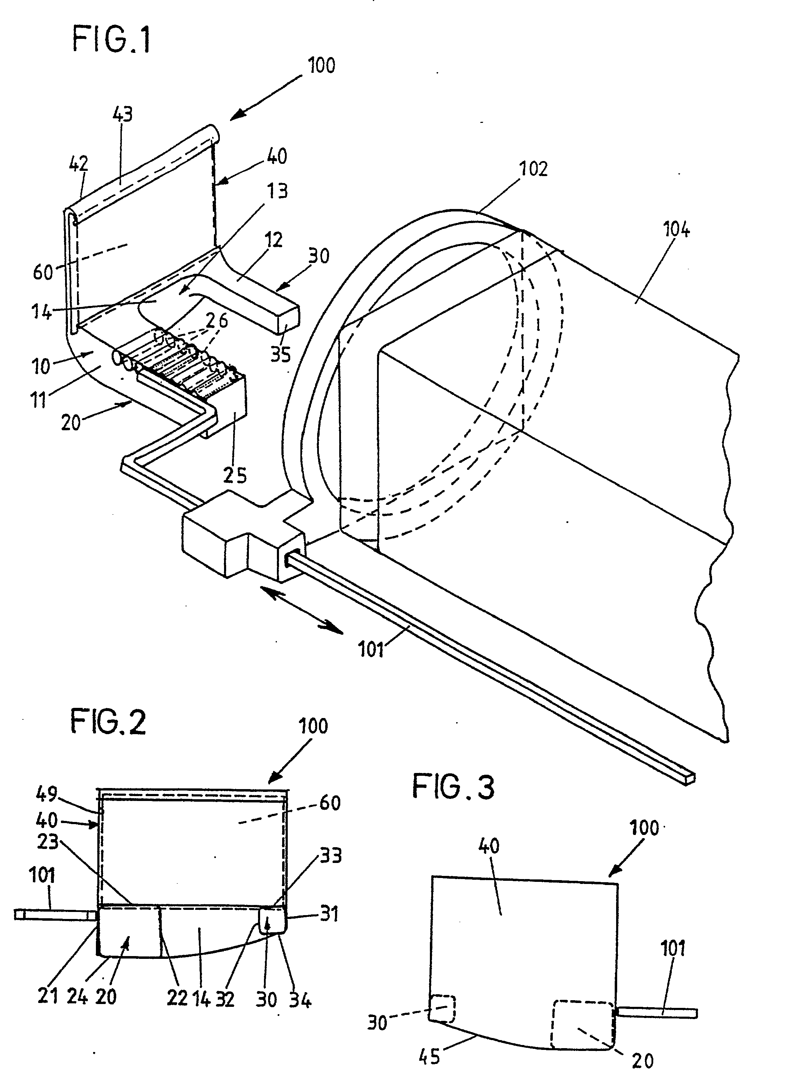

Fig. 1 zeigt einen Instrumentensatz für die Durchführung von Meßaufnahmen in der Endodontie in der Parallel-Technik, wobei dieser Instrumentensatz aus einem Filmhalter 100, einer mit diesem lösbar verbundenen Führungsstange 101, einem auf der Führungsstange 101 gehaltenen und in Führungsstangenlängsrichtung verschieblichen Visierring 102 und aus einem Langtubus 104 besteht, an dem der Filmhalter 100 vermittels der Führungsstange 101 und des Visierringes 102 befestigbar ist. Dieser Langtubus 104 ist Bestandteil eines in der Zeichnung nicht dargestellten zahnärztlichen Röntgengerätes, wobei dieser Langtubus 104 auch an ein Röntgengerät anschließbar sein kann.

Der Filmhalter 100 besteht aus einem etwa U-förmigen Formkörper 10 aus Kunststoffen, insbesondere aus Poly-Tetrafluoräthylen oder anderen geeigneten Kunststoffen oder Werkstoffen. Die beiden Schenkel des U-förmigen Formkörpers 10 sind mit 11, 12 und der die beiden Schenkel miteinander verbindende Steg mit 13 bezeichnet. Der Schenkel 11 ist als Aufbißblock 20, der Schenkel 12 als Kontakthalteblock 30 und der Steg 13 als Rückschild 40 ausgebildet, so daß der Filmhalter 100 von dem Aufbißblock 20, dem Kontakthalteblock 30 und dem Rückschild 40 zur Halterung eines Zahnfilmes 60 gebildet wird.

Der Kontakthalteblock 30 weist gegenüber der Länge des Aufbißblockes 20 eine kürzere Länge auf. Beide Blöcke 20, 30 sind parallel zueinander verlaufend an dem Rückschild 40 angeformt. Sowohl der Aufbißblock 20 als auch der Kontakthalteblock 30 weisen etwa quadratische Querschnitte auf, wobei jedoch der Querschnitt des Aufbißblockes 20 gegenüber dem Querschnitt des Kontakthalteblockes 30 größer ist (Fig. 2).

Der Aufbißblock 20 selbst besteht aus einem quaderförmigen bzw. rechteckförmigen Formkörper mit etwa quadratischem Querschnitt. Die Außenwandfläche des Aufbißblockes 20 ist mit 21, seine Innenwandfläche mit 22, seine obere Wandfläche mit 23, seine untere Wandfläche mit 24 und sein freies Ende mit 25 bezeichnet. Der Aufbißblock 20, der zu dem Rückschild 40 senkrecht, d.h. in einem rechten Winkel zu dem Rückschild stehend ist, weist eine Anzahl von parallel zueinander und zu dem Rückschild 40 verlaufende Durchbohrungen 26, auf, die zur Aufnahme und Halterung der Führungsstange 101 dienen, die die in Fig. 1 dargestellte, abgewinkelte Ausführungsform aufweist. An dem dem Aufbißblock 20 zugekehrten Ende der Führungsstange 101 sind vorzugsweise zwei im Abstand voneinander angeordnete, in der Zeichnung nicht dargestellte Zapfen oder Stifte vorgesehen, die in die Durchbohrungen 26 in dem Aufbißblock 20 einführbar sind, um die Führungsstange 101 an dem Aufbißblock 20 zu halten und um eine Abstandsveränderung des Filmhalters 100 von dem Langtubus 104 vornehmen zu können.

Der Kontakthalteblock 30 besteht vorzugsweise aus einem stab- bzw. stegartigen Formkörper mit einem quadratischen Querschnitt und verläuft parallel zu dem Aufbißblock 20. Im unteren Bereich des Rückschildes 40 geht der Kontakthalteblock 30 in das Rückschild 40 über. Die Außenwandfläche des Kontakthalteblockes 30 ist mit 31, seine Innenwandfläche mit 32, seine obere Wandfläche mit 33, seine untere Wandfläche mit 34 und sein freies Ende mit 35 bezeichnet. Die Übergangsbereiche 15, 16 der senkrechten Innenwandflächen 22, 32 des Aufbißblockes 20 und des Kontakthalteblockes 30 zu der Innenwandfläche 14 des Steges 13 bzw. des Rückschildes 40 sind bogenförmig ausgebildet, wobei jedoch der Abstand zwischen dem Aufbißblock 20 und dem Kontakthalteblock 30 so bemessen ist, daß ausreichend Platz zur Aufnahme von Wurzelkanalinstrumenten gegeben ist. Jedoch auf der anderen Seite sind die Gesamtabmessungen des Filmhalters so gehalten, daß die äußeren Begrenzungen des Filmhalters vorgegeben werden durch das Rückschild 40 zur Aufnahme des Zahnfilmes 60.

Das Rückschild 40 weist eine quadratische oder rechteckförmige Fläche auf; seine Zahnfilmaufnahmefläche ist mit 49 bezeichnet und sein oberer Rand mit 42. Dieser obere Rand 42 des Rückschildes 40 ist in Richtung zu dem Aufbißblock 20 und zu dem Kontakthalteblock 30 abgebogen, und zwar unter Ausbildung einer von unten zugänglichen Zahnfilmhaltenut 44, wobei der abgebogene Abschnitt bei 43 in Fig. 1 angedeutet ist. Die obere Randausgestaltung ist in Verbindung mit der ausgebildeten Zahnfilmhaltenut 44 derart, daß ein an der Zahnfilmaufnahmefläche 49 des Rückschildes 40 angelegter Zahnfilm 60 mit seinem oberen Randabschnitt in die Zahnfilmhaltenut 44 eingreift und vermittels dieses abgebogenen Abschnittes 43 lagegesichert ist. Die Abmessungen des Rückschildes 40 sind etwas größer als die Abmessungen handelsüblicher Zahnfilme, so daß ein an dem Rückschild 40 gehaltener Zahnfilm 60 mit seinen umlaufenden oder seitlichen Rändern nicht über die seitlichen Ränder des Rückschildes 40 hinausragen.

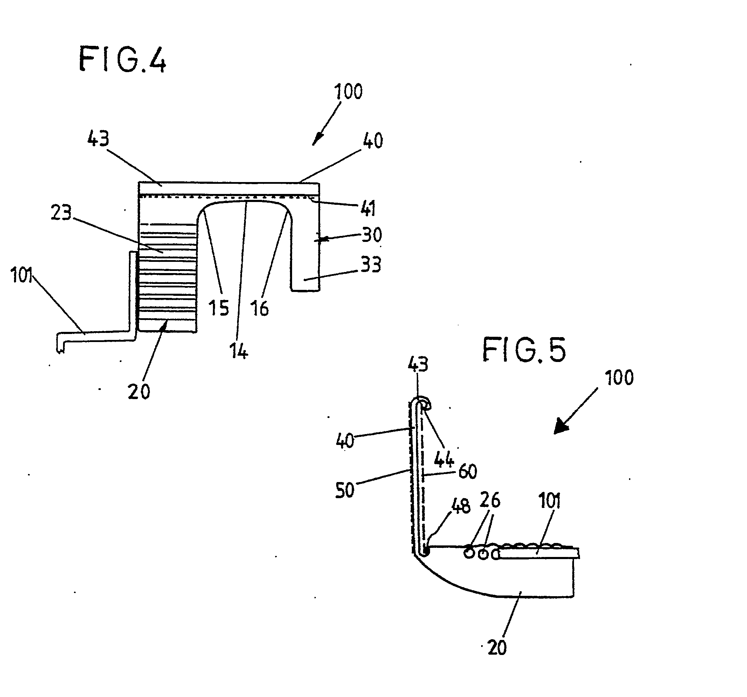

Im unteren Übergangsbereich des Rückschildes 40 zu den oberen Wandflächen 23, 33 des Aufbißblockes 20 und des Kontakthalteblockes 30 ist eine Zahnfilmauflagefläche 41 ausgebildet, die als durchgehender, z.B. in Richtung zu dem Aufbißblock 20 und dem Kontakthalteblock 30 vorspringender Absatz ausgebildet ist, der eine Breite aufweist, die in etwa der Breite eines handelsüblichen Zahnfilmes entspricht, so daß nach dem Anlegen eines Zahnfilmes 60 an das Rückschild 40 der Zahnfilm einerseits in der Zahnfilmhaltenut 44 des Rückschildes 40 gehalten ist und zum anderen sich gleichzeitig an der unteren Zahnfilmauflagefläche 41 abstützt, wobei diese Zahnfilmauflagefläche 41, die quasi die innenseitige untere Kante 45 des Rückschildes 40 bildet, auch als Rinne, Nut od.dgl. 48 ausgebildet sein kann (Fig. 5), um eine sichere Halterung des Zahnfilmes 60 in dem Filmhalter 100 zu gewährleisten. Das Einsetzen des Zahnfilms 60 in die Zahnfilmhaltenut 44 und in die rinnenförmige Zahnfilmauflagefläche erfolgt dann durch geringfügiges Abbiegen des Zahnfilms, wobei sich dann der Zahnfilm aus dem leicht abgebogenen Zustand nach dem Einsetzen in die Zahnfilmhaltenut 44 und die rinnenförmige Zahnfilmauflagefläche zurückbewegt, so daß der Zahnfilm plan an der Zahnfilmauflagefläche 41 des Rückschildes 40 anliegt.

Der Aufbißblock 20 und der Kontakthalteblock 30 bilden mit ihren oberen Wandflächen 23, 33 eine waagerechte Ebene, in der die Zahnfilmauflagefläche 41 des Rückschildes 40 liegt (Fig. 2). Der Aufbißblock 20 erstreckt sich im Bereich seiner bodenseitigen, d.h. unteren, Wandfläche 24 von seinem freien Ende 25 zu seinem rückwärtigen Bereich, d.h. zur unteren Kante 45 des Rückschildes 40 konisch verjüngend, wobei dann im rückwärtigen Bereich sich der untere Bereich des Rückschildes 40, wie aus Fig. 3 ersichtlich, in Richtung zum Kontakthalteblock 30 ebenfalls konisch verjüngt verläuft. Durch diese Erhöhung und Verlagerung des Aufbißblockes 20 nach mesial wird nicht nur ausreichend Platz zur Aufnahme der Wurzelkanalinstrumente geschaffen, sondern eine zusätzliche Stabilisierung am distalen Ende insofern erreicht, als bei eingesetztem Filmhalter der Kontakthalteblock 30 am distalen Ende für einen verbesserten Kontakt mit dem Gegenkiefer sorgt, was letztlich wiederum zu einem besseren Halt und einer exakteren Lage des Filmhalters im Mund des Patienten führt. Der Filmhalter erhält somit eine stabilere Lage, wenn ein entsprechender Aufbiß erfolgt. Der Filmhalter erhält neben dem Aufbißblock und dem Kontakthalteblock an dem distalen Ende im Rückschild einen zusätzlichen Abstützpunkt, so daß ein Verschieben des Filmhalters während der Meßaufnahmen vermieden wird.

Vorteilhafterweise ist das Rückschild 40 des Filmhalters 100 aus einem röntgenstrahlabsorbierenden Material gefertigt. Hierzu kann der für die Herstellung des Rückschildes 40 verwendete Kunststoff mit einer für Röntgenstrahlen schwer durchlässigen Substanz versehen sein, und zwar in Form von Beimischungen von Bariumsulfat, Gips, Wismutverbindungen od. dgl. Auch kann das Rückschild 40 mit einer röntgenstrahlabsorbierenden Beschichtung 50 versehen sein (Fig. 5). Diese röntgenstrahlabsorbierende Beschichtung 50 wird vorzugsweise an der rückwärtigen Außenwandfläche des Rückschildes 40 vorgesehen sein (Fig. 5). Diese Beschichtung 50 kann z.B. aus einer dünnen Bleiplatte bestehen, die jedoch auch in den Kunststoff eingearbeitet sein kann, aus dem das Rückschild besteht.

Die Abmessungen des Rückschildes 40 sind etwas größer als die Abmessungen des Zahnfilmes. Die Höhe des Rückschildes 40 entspricht dabei der Höhe des Zahnfilms, während die Breite des Rückschildes gegenüber der Breite des Zahnfilms größer ist.

Dadurch, daß der Zahnfilm 60 im oberen Bereich von dem abgebogenen Randabschnitt 43 des Rückschildes gehalten und im unteren Bereich sich gegen die Zahnfilmauflagefläche abstützt, steht die volle Zahnfilmfläche zu Meßaufnahmen zur Verfügung. Durch zusätzliche Klemmhalterung geht keine Zahnfilmfläche verloren.

## Patentansprüche

1. Filmhalter für endodontische Zahnaufnahmen in der Parallel-Technik, der aus einem als Formkörper (10) ausgebildeten Aufbißblock (20), einem an diesem angeformten und senkrecht zu der von dem Aufbißblock gebildeten Aufbißebene stehenden Rückschild (40) zur Halterung eines Zahnfilms, wobei der obere Rand (42) des Rückschildes (40) in Richtung zu dem Formkörper (10) unter Ausbildung einer Zahnfilmhaltenut (44) leicht abgebogen ausgebildet ist, einer mittels einer lösbaren Steckhalterung an dem Aufbißblock (20) gehaltenen Führungsstange (101) und einem auf dieser in Führungsstangenlängsrichtung verschieblichen Visierring (102) mit einer Halterung zum Befestigen des Filmhalters an dem Langtubus (104) eines zahnärztlichen Röntgengerätes, besteht, wobei das Rückschild (40) in eine dem Formkörper (10) zugekehrt angeordnete untere Zahnfilmauflagefläche (41) übergeht und gegenüber den Abmessungen des Zahnfilms größere Abmessungen aufweist, dadurch gekennzeichnet, daß der Formkörper (10) aus einem etwa U-förmigen Kunststoffteil besteht, dessen einer Schenkel (11) als Aufbißblock (20) und dessen anderer Schenkel (12) als Kontakthalteblock (30) ausgebildet ist, wobei der die beiden Schenkel (11, 12) miteinander verbindende Steg (13) den Rückschild (40) bildet, wobei der Kontakthalteblock (3) gegenüber der Länge des Aufbißblockes (20) eine kürzere Länge aufweist, daß der Aufbißblock (20) und der Kontakthalteblock (30) etwa quadratische Querschnitte aufweisen, wobei der Querschnitt des Aufbißblockes (20) gegenüber dem Querschnitt des Kontakthalteblockes (30) größer bemessen ist, daß die Übergangsbereiche (15, 16) der einander zugekehrten Wandflächen (22, 32) des Aufbißblockes (20) und des Kontakthalteblockes (30) zu der Wandfläche (14) des den Aufbißblock (20) mit dem Kontakthalteblock (30) verbindenden Steges (13) bogenförmig ausgebildet sind, daß die oberen Wandflächen (23, 33) des Aufbißblockes (20) und des Kontakthalteblockes (30) mit der unteren Zahnfilmauflagefläche (41) eine Ebene bilden, daß der Aufbißblock (20) sich bodenseitig (24) von seinem vorderen freien Ende (25) in Richtung zu dem Rückschild (40) konisch verjüngt und in die untere Kante (45) des Rückschildes (40) übergeht, die in die bodenseitige Wandfläche (34) des Kontakthalteblockes (30) übergeht, und daß das Rückschild (40) aus einem röntgenstrahlabsorbierenden Material besteht oder mit einer röntgenstrahlabsorbierenden Beschichtung (50) versehen ist.

2. Filmhalter nach Anspruch 1, dadurch gekennzeichnet, daß die Zahnfilmauflagefläche (41) rillenförmig ausgeildet ist.

## Revendications

1. Support de film pour prises de vues endodontiques de dents en technique parallèle, qui est constitué par un block pour mordre (20) configuré comme un corps moulé (10), par un bouclier arrière (40) moulé sur celui-ci et se tenant debout verticalement par rapport au plan pour mordre formé par le bloc pour mordre pour le maintien d'un film radiographique de dentiste, le bord supérieur (42) du bouclier arrière (40) étant configuré en étant légèrement recourbé en direction du corps moulé (10) en formant une rainure de retenue du film radiographique de dentiste (44), par une tige de guidage (101) maintenue sur le bloc pour mordre (20) au moyen d'un support emboîtable amovible et par un anneau devisée (102) translatable sur celle-ci dans le sens longitudinal de la tige de guidage avec un support pour la fixation du support de film au tube long d'un appareil de radiographie dentaire, le bouclier arrière (40) se poursuivant en une surface inférieure pour le dépôt du film radiographique de dentiste (41) disposée en étant tournée vers le corps moulé (10) et présentant des dimensions supérieures à celle du film radiographique de dentiste, caractérisé en ce que le corps moulé (10) est constitué par une partie en matière plastique approximativement en forme de U, dont l'une des branches (11) est formée comme bloc pour mordre (20) et dont l'autre branche (12) est formée comme bloc de maintien du contact (30), le dos reliant les deux branches l'une à l'autre formant le bouclier arrière (40), le bloc de maintien du contact (30) présentant une longueur inférieure à celle du bloc pour mordre (20), que le bloc pour mordre (20) et le bloc de maintien du contact (30) présentent des sections approximativement carrées, la section du bloc pour mordre (20) étant dimensionnée de façon plus grande que la section du bloc de maintien du contact (30), que les zones de transistion (15, 16) des faces de paroi (22, 32) tournées l'une vers l'autre du bloc pour mordre (20) et du bloc de maintien du contact (30) à la surface de la paroi (14) du dos (13) qui relie le bloc pour mordre (20) au bloc de maintien du contact (30) sont formées en arc, que les faces supérieures de paroi (23, 33) du bloc pour mordre (20) et du bloc de maintien du contact (30) forment un plan avec la surface inférieure pour le dépôt du film radiographique de dentiste (41), que le bloc pour mordre (20) s'effile sur sa face de fond (24) de son extrémité libre antérieure (25) en direction du bouclier arrière (40) et se convertit pour devenir l'arête inférieure (45) du bouclier arrière (40) qui devient la face de paroi (34) du côté du fond du bloc de maintien du contact (30) et que le bouclier arrière (40) est constitué par une matière qui absorbe les rayons X ou est pourvu d'un revêtement (50) qui absorbe les rayons X.

2. Support de film selon la revendication 1, caractérisé en ce que la surface pour le dépôt du film radiographique de dentiste (41) est configuré en forme de rainures.

**Claims**

1. Film holder for endodontic dental photographs in the parallel technique consisting of a bite block (20) constructed from a shaped member (10), a rear plate (40) formed on to the same assuming a vertical angle to the bite plane formed by the bite block for mounting a dental film, in which the upper rim (42) of the rear plate (40) is constructed so as to be slightly bent aside in the direction of the shaped member (10) while forming a dental film retaining groove (44), a guide rod (101) and a sight ring (102) which is displaceable on the latter in the direction of the guide rod with a mounting means for attaching the film holder on the long tube (104) of a dental X-ray apparatus, in which the rear plate (40) passes into a lower dental film bearing surface (41) and, as compared with the dimensions of the dental film, has larger dimensions, characterized in that the shaped member (10) consists of an approximately U-shaped plastic part, one of the legs (11) of which is constructed so as to form the bite block (20) and whose other leg (12) is constructed so as to form the contact retention block (30), in which the web (13) interconnecting the two legs (11, 12) forms the rear plate (40), in which the contact retention block (30), as compared with the length of the bite block (20), is shorter in length, in that the bite block (20) and the contact retention block (30) possess approximately square cross-sections, the cross-section of the bite block (20), as compared with the cross-section of the contact retention block (30), being dimensioned so as to be larger, in that the transitional areas (15, 16) of the wall surface areas (22, 23) of the bite block (20) and of the contact retention block (30) that face each other are, towards the wall surface (14) of the web (13) which connects the bite block (20) with the contact retention block (30), constructed so as to be curved, in that the upper wall surface areas (23, 33) of the bite block (20) and of the contact retention block (30) form one plane with the lower dental film bearing surface (41), in that the bite block (20), at the bottom (24), tapers conically from its forward free end (25) in the direction to the rear plate (40) and merges into the lower edge (45) of the rear plate (40) which, in turn, passes into the bottom wall surface (34) of the contact retention block (30), and in that the rear plate (40) consists of an X-ray absorbing material or is provided with an X-ray absorbing coating (50).

2. Film holder according to Claim 1, characterized in that the dental film bearing surface (41) is constructed so as to form grooves.

# FIG.1

# FIG.2

# FIG.3

# FIG.4

# FIG.5